(19) Europäisches Patentamt
European Patent Office
Office européen des brevets

(11) Publication number: 0 017 284
A2

(12) **EUROPEAN PATENT APPLICATION**

(21) Application number: 80200262.6

(22) Date of filing: 21.03.80

(51) Int. Cl.[3]: C 07 C 45/67
C 07 C 49/203, C 07 C 67/30
C 07 C 69/738, C 07 C 51/38
C 07 C 59/76

(30) Priority: 03.04.79 GB 7911678

(43) Date of publication of application:
15.10.80 Bulletin 80/21

(84) Designated Contracting States:
BE CH DE FR GB NL

(71) Applicant: SHELL INTERNATIONALE RESEARCH MAATSCHAPPIJ B.V.
Carel van Bylandtlaan 30
NL-2596 HR DEN HAAG(NL)

(72) Inventor: de Jong, Aaldert Johannes
Badhuisweg 3
NL-1031 CM Amsterdam(NL)

(72) Inventor: van Seters, Antonius Johannes Cornelis
Badhuisweg 3
NL-1031 CM Amsterdam(NL)

(74) Representative: Keuzenkamp, Abraham et al,
c/o Shell Internationale Research Maatschappij B.V. P.O. Box 302
NL-2501 CH 's-Gravenhage(NL)

(54) **Preparation of unsaturated ketones.**

(57) A process for the preparation of unsaturated ketones, in particular 5-hexen-2-one, by reacting a beta-keto ester and an allylhalide in the presence of an alkalimetal carbonate, and optionally a solvent, followed by hydrolysing the unsaturated keto ester obtained and decarboxylating the unsaturated keto acid formed.

EP 0 017 284 A2

Croydon Printing Company Ltd.

# PREPARATION OF UNSATURATED KETONES

The present invention relates to a process for the preparation of unsaturated ketones which are of interest as intermediates in the preparation of perfumes and/or vitamins. The present invention relates in particular to a process for the preparation of 5-hexen-2-one (allylacetone).

It is known from Japanese Published Patent Application No. 77 25709 that allyl- and benzyl acetones can be obtained by reacting alkylacetoacetates with allylic or benzylic halides in water in the presence of alkalimetal hydroxides followed by hydrolsis and decarboxylation. Although the yields quoted are rather high the process suffers from the serious drawback that the final product has to be recovered using a steam-distillation step which is laborious and energy consuming. Moreover, the reaction mixture comprises a high amount of water which also contains an additional amount of lithium hydroxide.

It is further known from Japanese Published Patent Application No. 74 26605 that allylacetone can be prepared from acetone and allylchloride using a large molar excess of acetone (more than 10 moles per mole of allylchloride) in the presence of a substantial amount of caustic alkali (1.0 - 3.0 mole) and a small amount of sodium iodide. In repeating the experiments it was found that the reaction product contains a substantial amount of the unwanted product mesityloxide as well as of higher condensation products up to an amount as high as fifteen times the amount of initially produced allylacetone. Again, the high ratio of solvent/reagent required is highly undesirable in a commercial process.

Although the use of an anhydrous alkalimetal carbonate is known for alkylation reaction in general (see, for instance J.Org.Chem.,Vol.43,No. 24,1978, pages 1682-4) it appears that virtually no reaction occurs when acetone and allylchloride are

heated under reflux conditions, even in the presence of a small amount of sodium iodide or a phase transfer catalyst.

From Dutch published patent application No. 72 16590 it is known that diketo esters can be obtained by reacting beta-oxo esters and alph-haloketones in solution in the presence of an alkalimetal carbonate and/or a tertiary amine. However, even when using the rather reactive alpha-haloketones large amounts of solvents are still required.

It has now been found that unsaturated ketones, and in particular 5-hexen-2-one can be prepared by reacting a beta-keto ester and an allylhalide in the presence of an alkali metal carbonate, optionally in the presence of a solvent, followed by hydrolysing the unsaturated keto ester obtained and decarboxylating the unsaturated keto acid formed.

The present invention therefore relates to a process for the preparation of unsaturated ketones according to the general formula:

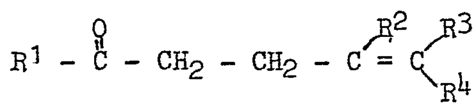

(I)

wherein $R^1$ represents an alkyl, cycloalkly, aryl, aralkyl or alkaryl group, and $R^2$,$R^3$ and $R^4$, each of which may be the same or different represent a hydrogen atom or an alkyl, cycloalkyl, aryl, aralkyl or alkaryl group or $R^3$ and $R^4$ together represent a tetra- or pentamethylene group, which comprises reacting a compound of the general formula:

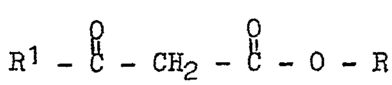

(II)

wherein $R^1$ has the meaning as defined hereinabove and R represents an alkyl group or a cycloalkyl group, with a compound according to the general formula:

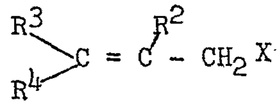

(III)

wherein $R^2$,$R^3$ and $R^4$ have the meanings as defined hereinabove and X represents a chlorine, bromine or iodine moiety in the presence of an alkalimetal carbonate followed by hydrolysing the unsaturated keto ester obtained and decarboxylating the unsaturated keto acid formed.

The process according to the present invention relates in particular to a process for the preparation of unsaturated ketones according to the general formula I by reacting a compound according to the general formula II in which $R^1$ represents an alkyl group of up to 10 carbon atoms or a cycloalkyl group having of from 5 to 7 carbon atoms or an aryl , aralkyl or alkaryl group of up to 10 carbon atoms and R represents a linear branched alkyl group of up to 10 carbon atoms or a cycloalkyl group of from 5 to 7 carbon atoms with a compound according to the general formula III wherein $R^2$,$R^3$ and $R^4$ each of which may be the same or different represent a hydrogen atom or an alkyl group of up to 10 carbon atoms, or a cycloalkyl group of from 5 to 7 carbon atoms or an aryl, alkaryl or alkaryl group of up to 10 carbon atoms and X represents a chlorine, bromine or iodine moiety in the presence of an alkalimetal carbonate followed by hydrolysing the unsaturated keto ester obtained and decarboxylating the unsaturated keto acid formed.

The present invention relates preferably to a process for the preparation of unsaturated ketones according to the general formula I by reacting a compound according to the general formula II in which $R^1$ represents an alkyl group of up to 4 carbon atoms, for instance a methyl, ethyl or isopropyl group; and R represents a linear or branched alkyl group of up to 6 carbon atoms, for instance a methyl, ethyl or isobutyl group with a compound according to the general formula III in which $R^2$,$R^3$ and $R^4$ each of which may be the same or different represent a hydrogen atom or an alkyl group of up to 6 carbon atoms, such

as a methyl, ethyl or isopropyl group and X represents a chlorine moiety in the presence of sodium and/or potassium carbonate and hydrolysing the unsaturated keto ester obtained and decarboxylating the unsaturated keto acid formed.

The present invention relates most preferably to a process for the preparation of 5-hexen-2-one by reacting allylchloride and (m)ethyl-3-oxo-butanoate in the presence of potassium carbonate followed by hydrolysing the product 2-acetyl-penten-5-oate which is then decarboxylated.

The halogen atom X in the general formula III - i.e. a chlorine, bromine or iodine moiety - is preferably a chlorine atom. Very good results have been obtained using allylchloride as the compound according to formula III.

The starting compounds having the general formulae II and III may be used in any desired ratio. Favourable molar ratios between the starting compounds according to the general formulae II and III are usually between 1:1 and 10:1, in particular between 1:1 and 5:1.

Of the alkalimetal carbonates which can be used - those of lithium, sodium, potassium, rubidium and cesium - those of potassium, rubidium and cesium are preferred. Special preference, because of its low price, is given to potassium carbonate.

It will be appreciated that the alkalimetal present in the system is converted with the hydrogen halide liberated during the present process into alkalimetal bicarbonate and alkalimetal halide. When the alkalimetal carbonate has been used up and hydrogenhalide is still being liberated, the alkali metal bicarbonate is converted with the latter hydrogen halide into alkali metal halide, carbon dioxide and water. It is preferable to keep the reaction medium anhydrous since water has a retarding effect on the first step of the present process. It is recommended to use a molar ratio of at least 1 and preferably between 1:1 and

2:1 between the alkali metal carbonate and the compound according to the general formula III.

The smaller the size of the alkali metal carbonate particles, the less chance there is, if molar ratios between the alkali metal carbonate and the compound according to the general formula III in excess of 1 are used, of formed alkali metal bicarbonate and hydrogen halide forming water; the smaller the particle size of the remaining alkali metal carbonate, the more readily it is accessible to hydrogen halide. The formation of unsaturated keto ester therefore takes place all the more rapidly and the selectivity to unsaturated keto ester is accordingly all the higher as alkali metal particles of smaller dimensions are used. Alkali metal carbonate particles of which the largest dimension is smaller than 0.2 mm are very useful. Particles of which the largest dimension is larger than 0.2 mm, for example between 0.5 mm and 2.5 mm, can be employed, but preferably in combination with the use of molar ratios of between, for example 2:1 and 5:1 between alkali metal carbonate and compound according to the general formula III. The use of potassium carbonate is preferred for economic reasons.

The process according to the present invention can be carried out in the presence or in the absence of a solvent. It has been found that it may be advantageous to add an inert solvent prior to the reaction or during the course of the reaction which is carried out by just mixing the ingredients, in order to prevent serious stirring problems. A reduced stirring, however, does not contribute adversely against the yield of the reaction concerned.

Suitable solvents which can be used in the process according to the present invention comprise ketones, ethers and hydrocarbons. Ketones are preferred . Examples of ketones are acetone, 2-butanone, 2-pentanone, 3-pentanone, 3-methyl-2-butanone, 3-methyl-2-pentanone and 2-hexanone. Acetone is

especially preferred. Examples of ethers which can be suitably applied as solvents comprise dimethoxyethane, diethoxy ethane, dioxane, dimethyl ether, diethyl ether and tetrahydrofuran whereas hydrocarbon solvents are examplified by benzene, toluene, ethylbenzene and the xylenes. Also mixtures of solvents can be suitably applied.

It is advisable to select the temperature of the reacting mixtures in such a way that the unsaturated ketone formation takes place at a reasonable rate. It has been found that temperatures between 50°C and 100°C give use to optium results. Lower as well as higher temperatures can be applied.

It is preferred that the process according to the present invention is to be carried out by thoroughly mixing the ingredients. This may be achieved, for example, by means of a stirring device, e.g. a propeller mixer and/or by refluxing the solution. Stirring is especially preferred when working in the absence or in the presence of a minor amount of a appropriate solvent. As a rule the reaction proceeds readily at atmospheric pressure, but super-atmospheric or sub-atmospheric pressures can be used as well.

It is advisable to carry out the process according to the present invention in the presence of a trace of an inorganic iodide such as sodium-, potassium or ammonium iodide, because inorganic iodides tend to increase the rate of unsaturated ketone formation. Preference is given to the use of sodium iodide, preferably in a molar ratio of compound according to formula III to iodide of between 100:1 and 1000:1. The latter range is not critical.

As referred to hereinabove, the concentrations in which the compounds having the formula II and III are used are not critical and may vary between wide limits. The concentrations to be chosen in a given case naturally depend on the solubility of the starting compound(s) in the solvent and may be, for example,

between 0.5 and 4.0 mole.

It has been found that the process according to the present invention can be carried out in the absence of a solvent at a much higher speed in the additional presence of a phase transfer catalyst which substantially obviates the use of an inorganic halide. Moreover, no excess of the compound according to formula II on the compound according to formula III is required. Suitable phase transfer catalysts comprise quaternary "onium" salts such as quaternary ammonium or sulphonium compounds as well as 18-crown-6- or related compounds. Very good results can be obtained using quaternary ammonium salts such as tetra-alkylammonium chlorides or bromides, e.g. aliquat, or tetrabutylammonium bromide as well as trimethyl-benzyl- or trimethylphenyl ammoniumchloride. The quaternary "onium" compounds can be applied in amounts similar to the inorganic iodine compounds.

The process according to the present invention can be carried out batchwise as well as continuously or semi-continuously.

The working up of the resultant reaction product which comprises as main product an unsaturated keto ester and converting it into a compound according to general formula I may be carried out in a conventional manner. In general, remaining alkali metal carbonate and salts formed from the alkali metal carbonate are first removed, e.g. by filtering or centrifuging. The solvent, if any and any starting materials which may still be present may subsequently be removed, e.g. by means of distillation, if desired at subatmospheric pressure.

The unsaturated keto ester obtained can be converted into the corresponding compound according to formula I by methods known in the art; e.g. by converting the unsaturated keto ester with an alkali solution into the corresponding alkali salt which is decarboxylated under the reaction conditions using for instance hydrochloric acid or sulphuric acid to give the final

compound according to formula I. Aqueous sodium hydroxide in concentrations up to about 30%, preferably in the range of from 5-15% can be suitably applied in the saponification step.

The invention will now be illustrated by means of the following Examples.

Example I

0.1 Mole of allylchloride was added slowly to a solution boiling under reflux of 0.2 mole of methylacetoacetate (MAA) in 100 ml of dry acetone to which 0.1 mole of anhydrous potassium carbonate and 0.05 g of sodium iodide had been added. The reaction was allowed to proceed for 20 hours. After cooling down the reaction mixture a 5% aqueous NaOH-solution was added in order to saponify the ester into the unsaturated keto acid which was then acidified with HCl followed by extracting the aqueous layer with diethylether which gave crude 5-hexen-2-one (allyl acetone) in 65% isolated yield. Distillation under reduced pressure afforded 5-hexen-2-one of 99% purity.

Example II

The experiment described in Example I was repeated with the exception that 0.3 mole of methylacetoacetate (MAA) and 0.2 mole of allylchloride were used. After working up 5-hexen-2-one appeared to have been formed (69.5% calculated on allychloride). Repeating the experiment using 0.25 mole of MAA per 0.2 mole of allylchloride afforded 74.4% of 5-hexen-2-one together with a small amount of dicondensation product.

Example III

The experiment described in Example I was repeated with the exception that 0.3 mole of ethylacetoacetate and 0.2 mole of allylchloride were used. After working up 5-hexen-2-one had been formed(54.0% calculated on allylchloride) together with about 1% of dicondensation product.

Example IV - Preparation of 5-hexen-2-one in the absence of a solvent.

0.1 Mole of allylchloride and 0.1 mole of MAA were heated at 70°C in the absence of any solvent but in the presence of a tiny amount of tetramethylammonium chloride (aliquat) - which can be replaced equally well with trimethyl benzyl ammonium chloride - for 1¼ hours. After working up the reaction mixture 5-hexen-2-one had been formed (76% calculated on allylchloride) together with a small amount of a dicondensation product. As the absence of a solvent caused a rather cumbersome working up procedure, the experiment was repeated but this time omitting also the phase transfer catalyst but adding 100 ml of acetone when stirring problems started to arise. Similar results were obtained after prolonged reaction time.

Example V - Preparation of 5-hexen-2-one in the presence of toluene

The experiment described in Example I was repeated, this time replacing the solvent acetone with the same amount of toluene which also contained a very small amount of trimethylbenzylammonium chloride. After 16 hours the reaction mixture was worked up and 5-hexen-2-one had been formed (70% calculated on allylchloride) together with only 2% of dicondensation product.

Comparative Examples

(A) Instead of using MAA or ethylacetoacetate (EAA) acetone was used as the reagent as well as the solvent in the presence of sodium hydroxide instead of potassium carbonate and a very small amount of sodium iodide. After 1 h a substantial amount of acetone-condensation products had been formed which were far in excess of the desired unsaturated keto ester.

(B) The comparative experiment as described in Comparative Example A was repeated using anhydrous potassium carbonated and a very small amount of sodium iodide. Virtually no reaction occurred.

(C) The comparative experiment as described in Comparative Example B was repeated in the additional presence of a phase

transfer catalyst (trimethylbenzyl ammonium chloride). Virtually no reaction could be detected at all.

# C L A I M S

1. A process for the preparation of unsaturated ketones according to the general formula:

$$R^1 - \overset{\overset{O}{\|}}{C} - CH_2 - \overset{R^2}{C} = C\begin{matrix} R^3 \\ \\ R^4 \end{matrix} \qquad (I)$$

wherein $R^1$ represents an alkyl, cycloalkyl, aryl, aralkyl or alkaryl group, and $R^2$,$R^3$ and $R^4$, each of which may be the same or different, represent a hydrogen atom or an alkyl, cycloalkyl, aryl, aralkyl or alkaryl group or $R^3$ and $R^4$ together represent a tetra- or pentamethylene group, which comprises reacting a compound of the general formula:

$$R^1 - \overset{\overset{O}{\|}}{C} - CH_2 - \overset{\overset{O}{\|}}{C} - O - R \qquad (II)$$

wherein $R^1$ has the meaning as defined hereinabove and R represents an alkyl group or a cycloalkyl group, with a compound according to the general formula:

$$\begin{matrix} R^3 \\ \\ R^4 \end{matrix} C = \overset{R^2}{C} - CH_2X \qquad (III)$$

wherein $R^2$, $R^3$ and $R^4$ have the meanings as defined hereinabove and X represents a chlorine, bromine or iodine moiety, in the presence of an alkalimetal carbonate followed by hydrolysing the unsaturated keto ester obtained and decarboxylating the unsaturated keto acid formed.

2. A process according to claim 1 in which a compound according to the general formula I is prepared by reacting a compound according to the general formula II in which $R^1$ represents an alkyl group of up to 10 carbon atoms or a cycloalkyl group having of from 5 to 7 carbon atoms or an aryl, aralkyl or alkaryl group of up to 10 carbon atoms and R represents a linear or branched alkyl group of up to 10 carbon atoms or a

cycloalkyl group of from 5 to 7 carbon atoms, with a compound according to the general formula III wherein $R^2$,$R^3$ and $R^4$ each of which may be the same or different, represent a hydrogen atom or an alkyl group of up to 10 carbon atoms, or a cycloalkyl group of from 5 to 7 carbon atoms or an aryl, aralkyl group of up to 10 carbon atoms and X represents a chlorine, bromine or iodide moiety in the presence of an alkalimetal carbonate followed by hydrolysing the unsaturated keto ester obtained and decarboxylating the unsaturated keto acid formed.

3. A process according to claim 2 in which a compound according to the general formula I is prepared by reacting a compound according to the general formula II in which $R^1$ represents an alkyl group of up to 4 carbon atoms, for instance a methyl-, ethyl-, or isopropyl group, and R represents a linear or branched alkyl group of up to 6 carbon atoms, for instance a methyl, ethyl or isobutyl group, with a compound according to the general formula III in which $R^2$,$R^3$ and $R^4$, each of which may be the same or different, represent a hydrogen atom or an alkyl group of up to 6 carbon atoms, such as a methyl, ethyl- or isopropyl group, and X represents a chlorine moiety in the presence of sodium and/or potassium carbonate and hydrolysing the unsaturated keto ester obtained and decarboxylating the unsaturated keto acid formed.

4. A process according to claim 3 in which 5-hexen-2-one is prepared by reacting allylchloride and (m)ethyl-3-oxo-butanoate in the presence of potassium carbonate followed by hydrolysing the product 2-acetyl-penten-5-oate which is then decarboxylated.

5. A process according to any one of the preceding claims, wherein use is made of allylchloride as the compound according to the general formula III.

6. A process according to any one of the preceding claims, wherein the ratio between the starting compounds according to the general formulae II and III is between 1:1 and 10:1,

in particular between 1:1 and 5:1.

7. A process according to any one of the preceding claims, wherein the alkalimetal carbonate used is potassium carbonate.

8. A process according to any one of the preceding claims, wherein the alkalimetal carbonate and the compound according to the general formula III are used in a molar ratio of at least 1, preferably between 1:1 and 2:1.

9. A process according to any one of the preceding claims, wherein the alkalimetal carbonate is used in particles of which the largest diameter is smaller than 0.2 mm.

10. A process according to any one of the preceding claims, which comprises carrying out the reaction in the presence of a solvent.

11. A process according to claim 10, wherein a ketone, preferably acetone or an aromatic compound, preferably toluene, is used as the solvent.

12. A process according to any one of the preceding claims, wherein a reaction temperature is applied between 50°C and 100°C.

13. A process according to any one of the preceding claims, wherein a small amount of an inorganic iodide, preferably sodium iodide, is also present in the reaction mixture.

14. A process according to any one of claims 1-12, wherein the process is carried out in the additional presence of a phase transfer catalyst.

15. A process according to claim 14, wherein the phase transfer catalyst applied is a tetraalkylammonium halide, preferably tetramethylammonium chloride or a trialkylbenzylammonium halide, such as trimethylammonium chloride.